# EUROPEAN PATENT SPECIFICATION

(11) **EP 4 173 002 B1**
(45) Date of publication and mention of the grant of the patent: **09.10.2024**
(21) Application number: 21740200.7
(22) Date of filing: 22.06.2021
(51) Int. Cl.: G16H 40/20, G16H 40/63

(54) **MONITORING SYSTEM OF BLOOD COMPONENTS BAGS**
ÜBERWACHUNGSSYSTEM FÜR BLUTKOMPONENTENBEUTEL
SYSTÈME DE SURVEILLANCE DE POCHES DE COMPOSANTS SANGUINS

(30) Priority: 26.06.2020 IT 202000015538
(43) Date of publication of application: 03.05.2023
(73) Proprietor: IDEA SOC. COOP., 60131 Ancona (AN) (IT)
(72) Inventor: DI BUO', Gianluca, 60131 Ancona (AN) (IT)
(74) Representative: Grana, Daniele
(86) International application number: PCT/IB2021/055477
(87) International publication number: WO 2021/260534

(56) References cited:
- EP-A1- 2 436 361
- EP-B1- 2 436 361
- WO-A2-2014/081839
- US-A1- 2019 336 706

## Description

### Technical Field

The present invention relates to a monitoring system of blood component bags.

### Background Art

In the field of transfusion medicine, but also in the administration of drugs by intravenous infusion, the use of special bags is known which can be connected to infusion means, such as e.g. needles, for the infusion of their contents into the bloodstream of a patient.

In particular, in the specific case of blood components, the bags are previously provided with mixtures of anticoagulants and preservative solutions adapted to preserve the vitality and functionality of blood cells. Once filled, in the so-called blood banks, the bags are stored at refrigerated temperatures for more than one month or can be frozen for longer periods.

At the time of use, bags must be transported under refrigerated conditions from the blood bank to a destination facility in order to preserve the above characteristics until infusion. It is easy to understand that interruptions in the cold chain, even if of short duration, can lead to alterations in the contents of the bags.

For this reason, it is well known to use bags provided with temperature sensors, able to detect and monitor the temperature values to which each bag is subjected.

Such bags are generally made with a sensor built into them and, once used, are disposed of together with the sensor itself.

It is clear, therefore, that this solution is decidedly impractical and expensive compared to traditional bags and can involve very high costs for the facilities responsible for the collection. Not least, according to current regulations, the blood component bags must necessarily be disposed of as special waste. The electronic components, which make up the sensors associated with them, cannot, therefore, be properly differentiated, with a consequent high environmental impact.

In addition, the sensors used are not capable of detecting other physical entities or other identifying data useful in correctly associating the bag with a particular patient.

In this regard, it is clear that it is of paramount importance that the bags are delivered to the correct patient. In fact, administering blood types to an incompatible patient can be fatal.

In addition, doubts about the origin of the bag, the conditions to which it was subjected during transport, or the patient for whom it is intended may require disposal of the bag prior to use.

These issues are partially overcome by the monitoring system described in document WO 2017/136866A1.

WO 2017/136866A1 describes a monitoring system for biological products provided with a sensing device configured to record bag temperature values and identifying data such as identifying data about the bag, its contents, the destination transfusion center, the assigned patient, and the like.

At the time of the blood component collection, the sensing device is inserted into a special pocket formed in the bag, or applied to the bag by adhesive means and, following patient administration, it is removed before disposal for reuse. The monitoring system described by WO 2017/136866A1 does however have some drawbacks.

In fact, such a monitoring system does not ensure secure bag tracking. The sensing device may accidentally fall out of the pocket or be removed, or it can be applied to a different bag without any possibility for the operator to notice it. It follows that the aforementioned system does not allow monitoring in an unequivocal way the environmental conditions to which the bag is subjected, nor does it allow verifying that the bag is intended for a specific patient, with the risk of incurring the problems discussed above.

Documents EP 2 436 361 A1, WO 2014/081839 and US 2019/336706 disclose devices forming part of the close prior art.

### Description of the Invention

The main aim of the present invention is to devise a monitoring system of blood component bags which allows a blood component bag to be unambiguously monitored and which ensures proper and safe association between the bag itself and a sensing device.

Another object of the present invention is to devise a monitoring system for blood component bags which will also detect temporary removal of the sensing device from the respective bag.

Still one object of the present invention is to devise a monitoring system for blood component bags which allows detecting a plurality of different environmental conditions to which the bag is subjected.

Another object of the present invention is to devise a monitoring system for blood component bags which allows overcoming the aforementioned drawbacks of the prior art within a simple, rational, easy and effective to use as well as affordable solution.

The aforementioned objects are achieved by the present monitoring system of blood component bags having the characteristics of claim 1.

### Brief Description of the Drawings

Other characteristics and advantages of the present invention will become more apparent from the description of a preferred, but not exclusive, embodiment of a monitoring system of blood component bags, illustrated by way of an indicative, yet non-limiting example, in the accompanying tables of drawings wherein:

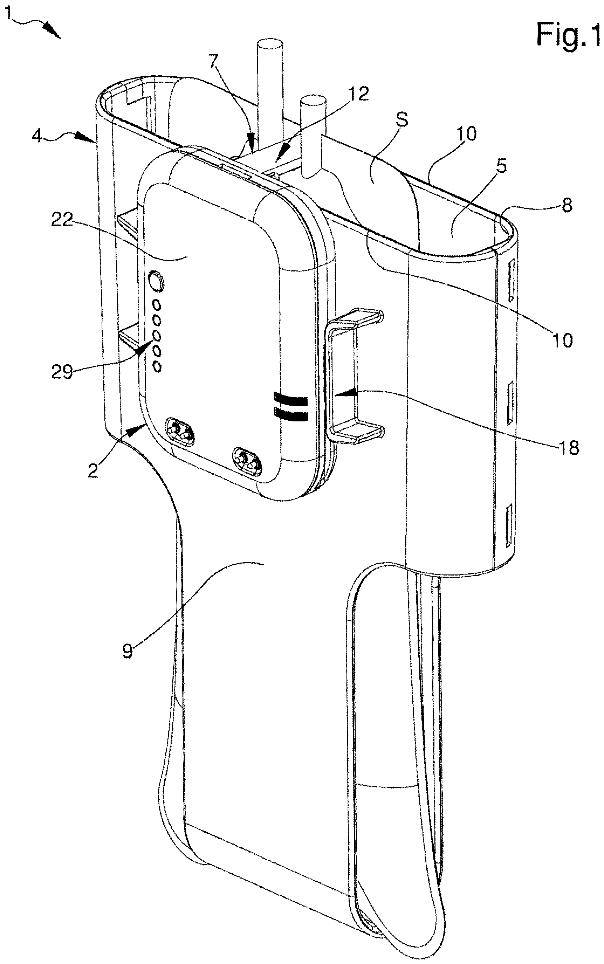

Figure 1 is an axonometric view of part of the monitoring system according to the invention, provided with a blood component bag;

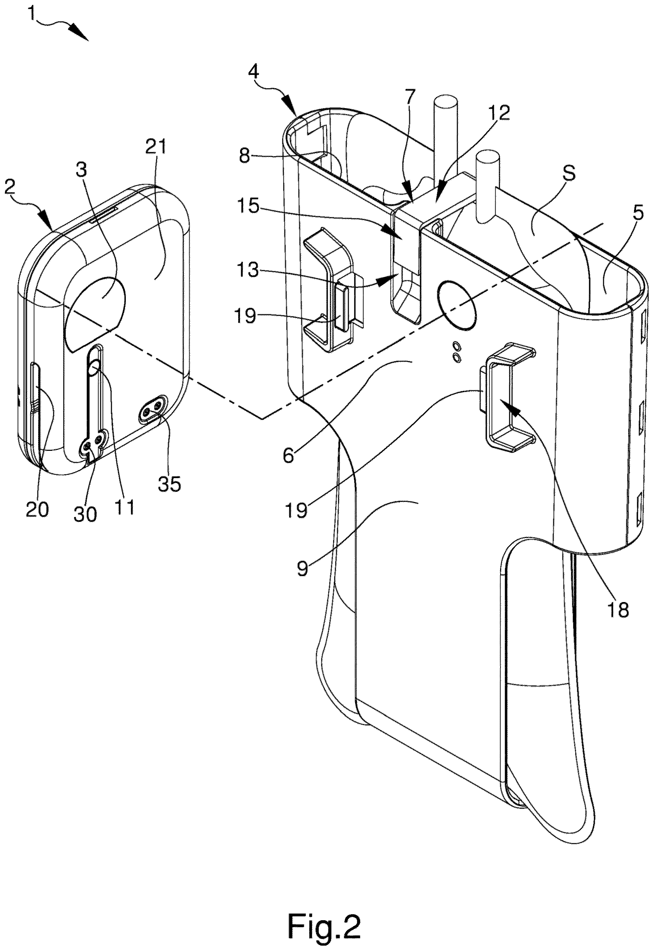

Figure 2 is an axonometric view of part of the monitoring system according to the invention, partly in exploded view;

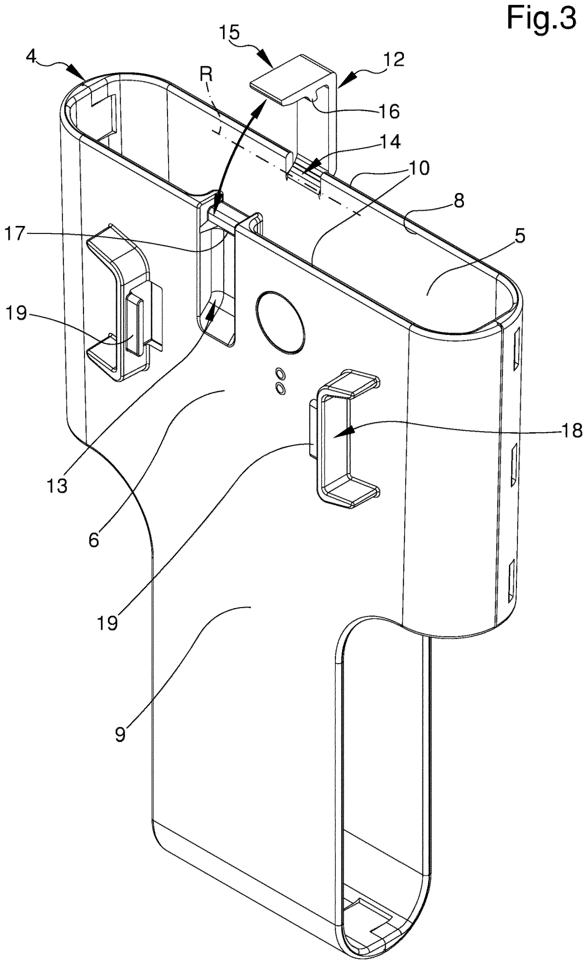

Figure 3 is a view of a support according to the invention, in open configuration;

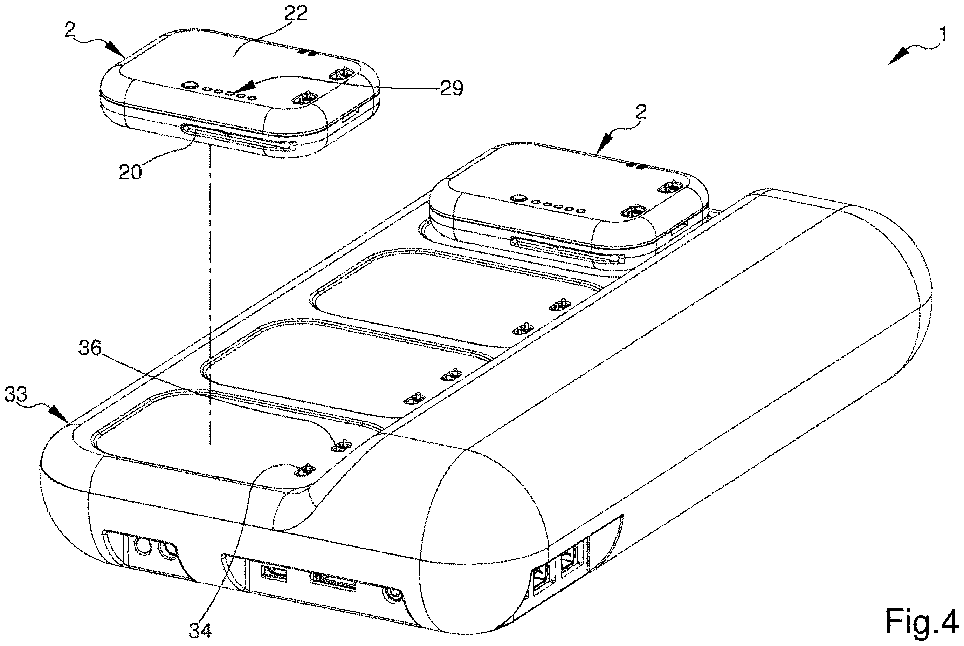

Figure 4 is an axonometric view of a charging station for sensing devices according to the invention;

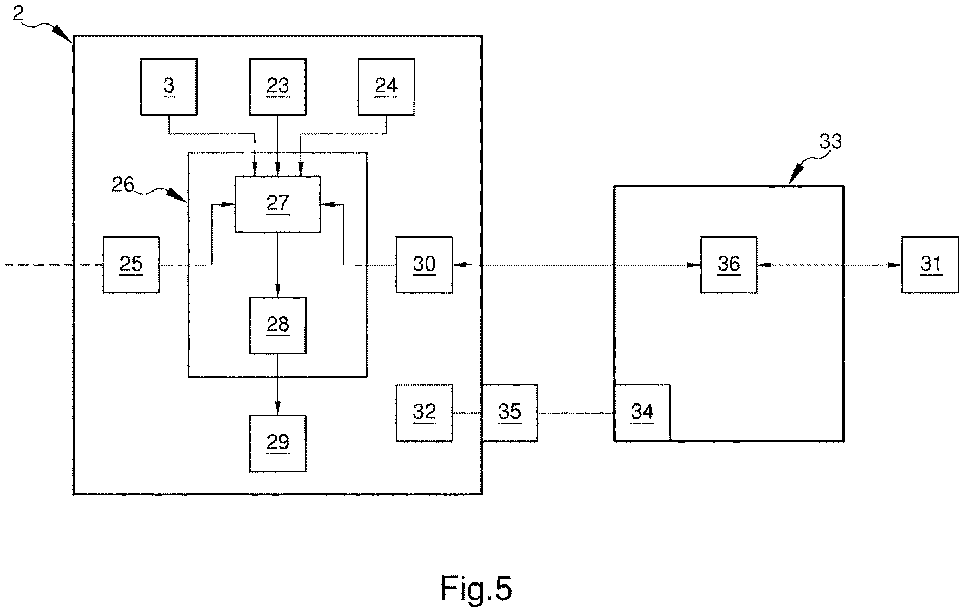

Figure 5 shows a block diagram of the monitoring system, according to the invention.

### Embodiments of the Invention

With particular reference to these figures, reference numeral 1 globally indicates a monitoring system of blood component bags.

The monitoring system 1 comprises at least one sensing device 2 comprising at least one temperature sensor 3 configured to detect at least one value of temperature of at least one bag S containing blood components.

In the present discussion, "bag" means a container consisting of a pair of sheets of polymeric material welded together around their perimeter edges to obtain an internal cavity, in which a product for intravenous infusion is inserted, in this case blood or blood components. It cannot, however, be ruled out that the present monitoring system may be applied to bags for the containment and transport of drugs for intravenous infusion or bags of different types.

The sensing device 2 is movable with respect to the bag S between an operating position, wherein it is positioned at the point where the bag S is located and wherein the temperature sensor 3 detects the value of temperature, and a far-away position, wherein it is moved away from the bag S.

The sensing device 2 is associable in a removable manner with the bag S so that it can be reused several times as a result of the infusion operations of the bag contents.

According to the invention, the monitoring system 1 is also provided with:
- at least one support 4 of the bag S comprising at least one housing 5 of the bag S and at least one seat 6 for housing the sensing device 2 in the operating position; and
- retaining means 7 of the bag S associated with the support 4 and positionable between an open configuration, wherein the bag S is movable with respect to the housing 5, and a closed configuration, wherein the bag S is retained inside the housing 5.

The support 4 comprises a transit opening 8 adapted to allow the insertion/extraction of the bag S into/from the support itself.

More in detail, according to a preferred embodiment, the support 4 comprises at least two opposite and mutually associated walls 9, preferably by interlocking, along their respective side edges to define the housing 5 between them. The walls 9 comprise at least two corresponding free side edges 10 delimiting the transit opening 8.

Substantially, through the transit opening 8, the bag S is positionable in the housing 5 and is retained inside the support 4 by means of the retaining means 7.

The sensing device 2 is associable with the support 4 in a removable manner. Furthermore, the sensing device 2 comprises at least one contact sensor 11 configured to detect the positioning of the sensing device 2 in the operating position.

The contact sensor 11 allows sensing and recording any removal of the sensing device 2 from the support 4 so as to ensure proper traceability of the bag S.

In this case, the contact sensor 11 is of the type of, e.g., a pressure sensor. Again, in the operating position, the sensing device 2 is configured to lock the retaining means 7 in the closed configuration.

In other words, when the sensing device 2 is associated with the support 4, in its own seat 6, the retaining means 7 cannot be moved to the open configuration and the bag S cannot be removed from the support. The removal of the sensing device 2 to allow extraction of the bag S is readily detected by the contact sensor 11.

The retaining means 7 comprise a retaining element 12 movable with respect to the support 4 between the open configuration and the closed configuration.

In detail, in the closed configuration, the retaining element 12 is shaped so as to at least partly obstruct the transit opening 8.

In the embodiment shown in the figures, the retaining element 12 is of a substantially elongated conformation.

The retaining element 12 is positionable to be arranged between the free side edges 10 so as to prevent the transit of the bag S through the transit opening 8. Conveniently, the support 4 comprises an abutment portion 13 which is shaped to accommodate the retaining element 12 in the closed configuration.

The abutment portion 13 is formed in at least one of the walls 9 at the point where the free side edge 10 is located.

Conveniently, the retaining element 12 comprises a first portion 14 associated with the support 4 and a second portion 15, opposite the first portion 14, shaped to engage with the abutment portion 13, in the closed configuration.

The retaining means 7 comprise fastening means 16, 17, of the interlocking type, positioned between the second portion 15 and the abutment portion 13.

In more detail, according to a possible embodiment, the fastening means 16, 17 comprise a protrusion 16 formed on the second portion 15 and shaped so as to mate with a corresponding recess 17 formed in the abutment portion 13.

The interaction between the protrusion 16 and the recess 17 results in a firm mating between the retaining element 12 and the abutment portion 13 and prevents accidental opening of the retaining means 7.

It cannot, however, be ruled out that the fastening means 16, 17 may be of a different type, e.g. of the type of a pair of small teeth of complementary conformation to each other.

Advantageously, the retaining element 12 is movable in rotation around a relevant axis of rotation R, at the point where the first portion 14 is located, between the open configuration and the closed configuration.

In more detail, the axis of rotation R extends parallel to one of the free side edges 10 and in the proximity thereof.

The first portion 14 is associated with the support 4 along the axis of rotation and is rotatable with respect thereto.

By rotating around the axis of rotation R towards the closed configuration, the second portion 15 reaches the abutment portion 13 and engages with the latter. Advantageously, the seat 6 of the sensing device 2 is defined at the point where the abutment portion 13 is located.

In other words, the seat 6 is obtained on the wall 9, on which the abutment portion 13 is defined, at the point where the relevant free side edge 10 is located.

In the operating position, the sensing device 2 is positioned at the point where the abutment portion 13 is located to prevent the retaining element 12 from rotating from the closed configuration towards the open configuration.

The positioning of the sensing device 2 in the seat 6 keeps, therefore, the second portion 15 engaged with the abutment portion 13.

This makes it impossible to remove the bag from the support 4 without first removing the sensing device from the seat 6.

Conveniently, the support 4 comprises anchoring means 18 of the sensing device 2 to the seat 6, of the interlocking type.

In more detail, the anchoring means 18 comprise a pair of sliding rails 19 formed on the wall 9 of the support 4 and adapted to fit into corresponding grooves 20 formed on the sensing device 2.

In the embodiment shown in the figures, the sliding rails 19 extend transversely to the free side edges 10.

The sensing device 2 is, therefore, positioned in the seat 6 by sliding along the sliding rails 19 until it reaches the operating position.

The sensing device 2 comprises an enclosure 21, 22 provided with a contact wall 21 facing a wall of the housing 5 at the point where the seat 6 is located, in the operating position and an external wall 22 facing outwards.

Conveniently, the temperature sensor 3 and the contact sensor 11 are arranged on the contact wall 21.

In this way, in the operating position, the temperature sensor 3 is arranged adjacent to the bag S and the contact sensor 11 is activated by the abutment with the wall of the support 4.

Advantageously, the sensing device 2 comprises at least one humidity sensor 23 configured to detect at least one value of humidity external to the bag S and at least one brightness sensor 24 configured to detect at least one value of brightness external to the bag S.

The presence of the humidity sensor 23 and of the brightness sensor 24 therefore allows sensing additional environmental parameters that could compromise the quality of the product contained in the bag S.

The sensing device 2 is capable of periodically recording these values so as to ensure near-constant monitoring.

The sensing device 2 also comprises acquisition means 25 configured to acquire at least one control datum.

In this case, the control datum is selected out of: an identification control datum of a patient or an identification control datum of at least one caregiver.

Preferably, the acquisition means 25 are configured to acquire at least one identification control datum of a patient.

The acquisition means 25 allow, therefore, recording at least one datum concerning the patient in order to ensure the correct and quick assignment of the bag S to the patient him/herself.

Conveniently, the acquisition means 25 are of the radio frequency type. Preferably, the acquisition means 25 are of the NFC (Near Field Communication) type.

The NFC transceiver technology is of a type known to the engineer of the industry and will not be described in detail in the present disclosure.

The monitoring system 1 comprises identification means of the NFC type, not shown in detail in the figures, configured to communicate a corresponding control datum to the acquisition means 25.

In this case, the identification means are of the type of a bracelet and/or a badge provided to the receiving facility for the patient and/or caregivers.

The sensing device 2 comprises at least one electronic unit 26 provided with a memory unit 27 configured to store at least one of either a value of temperature, a value of humidity, a value of brightness, at least one control datum or at least one preset datum.

In this case, the preset datum is selected out of: a preset datum identifying the bag S, a preset datum identifying the contents of the bag S, a preset datum identifying a patient, a preset datum identifying a destination of use of the bag S.

Substantially, the sensing device 2 allows storing a set of preset data, at the beginning of the monitoring operations of the bag S, and a set of control data, acquired by means of the acquisition means 25, during the transit of the bag S to the patient.

Similarly to the control data, the preset data are also loaded onto the memory unit 27 by means of the acquisition means 25.

In more detail, the monitoring system 1 comprises loading means, not shown in detail in the figures, configured to communicate the preset data to the acquisition means 25.

The loading means are of the type of an NFC writer.

The memory unit 27 is also configured to store values of position detected by the contact sensor 11. In other words, the memory unit 27 is able to store any removal of the sensing device 2 from the seat 6.

Conveniently, the electronic unit 26 comprises a processing unit 28 configured to check the matching between the preset datum and a corresponding control datum.

For example, the processing unit 28 is configured to compare the identification control datum of the patient receiving the bag S with the corresponding preset identification datum of the patient loaded on the memory unit 27 at the beginning of the monitoring operations.

The sensing device 2 also comprises indication means 29 configured to indicate a result of the check.

In the embodiment shown in the figures, the indication means 29 are of the luminous type and comprise one or more LED units, each corresponding to a control datum to be acquired.

The indication means 29 may be fixed and/or intermittent light.

It cannot however be ruled out that the indication means 29 are of different types. For example, the indication means 29 may be of an audible type or both of a luminous and an audible type.

Advantageously, the sensing device 2 comprises transmission means 30 operationally connected to the memory unit 27 and configured to transmit at least one of at least one of the values or at least one of the identification datum to at least one external unit 31.

The sensing device 2 allows transferring the acquired data to the external unit 31 in order to allow them to be stored on different electronic devices or to carry out an additional control.

According to a preferred embodiment, the transmission means 30 are of the Bluetooth type.

In combination or alternatively, the transmission means 30 may be of the CAN (Controller Area Network) type.

Bluetooth and CAN technologies are of a type known to the engineer in the industry and will not be described in detail in the present disclosure.

The sensing device 2 comprises storage means 32 for storing electrical energy which are configured to supply the sensing device 2.

The storage means 32 are of the type of a rechargeable battery. Advantageously, the monitoring system 1 comprises a charging station 33 configured to house at least one sensing device 2 and comprising at least electrical charging means 34 of the sensing device 2.

In turn, the electrical charging means 34 are connectable to an energy source, such as e.g. the power grid.

In the embodiment shown in the figures, the charging station 33 is configured to house a plurality of sensing devices 2.

The storage means 32 of each sensing device 2 comprise connecting elements 35 connectable to the electrical charging means 34.

When the sensing device 2 is in the charging station 33, the connecting elements 35 interact with the electrical charging means 34 to enable the transfer of electrical energy.

Conveniently, the charging station 33 comprises communication means 36 configured to receive at least one of at least one of the values or at least one of the control data from each of the sensing devices 2 and to transmit them to the external unit 31.

For this purpose, the transmission means 30 are connectable to the communication means 36.

The communication means 36 are also configured to acquire the preset data from the external unit 31 and to transfer them to the sensing devices 2.

The operation of the monitoring system of blood component bags according to the present invention is as follows.

At the beginning of the monitoring operations, the sensing device 2 is arranged in the charging station 33, with the connecting elements 35 in contact with the electrical charging means 34.

The bag S is inserted inside the support 4, into the housing 5, the retaining means 7 are brought to the closed configuration.

In more detail, the retaining element 12 is rotated around the axis of rotation R so that the second portion 15 comes into contact with the abutment portion 13. At this position, the curved protrusion 16 of the second portion 15 engages with the recess 17 of the abutment portion 13.

When the sensing device 2 is in the seat 6, the retaining means 7 cannot be brought to the open configuration again.

The contact sensor 11 detects the correct positioning of the sensing device 2 in the operating position.

In the event of the sensing device 2 being removed from its own seat 6, the contact sensor 11 detects this removal and stores it in the memory unit 27.

The sensors 3, 23, 24 begin the detection of their respective values and continue for the entire duration of the transit of the bag S towards a receiving facility. The loading means communicate to the sensing device 2, by means of the acquisition means 25, the preset data concerning the bag S, the destination of the bag S and the patient. The preset data are stored on the memory unit 27.

At this point, the bag S, inside the housing 5 is transferred towards the receiving facility where the identification means communicate to the sensing device 2, by means of the acquisition means 25, at least one identification control datum of the patient and the processing unit 28 checks the correspondence thereof with the relevant preset datum.

The indication means 29 indicate to an operator the outcome of such a check, and if successful, the bag S is administered to the patient.

During such operations, the sensing device 2 may also acquire and store the control data identifying the caregivers involved.

Upon completion of infusion of the contents of the bag S, the sensing device 2 is removed from the support 4 to allow the disposal of the bag S.

The sensing device 2 is, then, connected to the charging station 33 to allow the acquired control data to be transferred and stored by the external unit 31.

It has in practice been ascertained that the described invention achieves the intended objects, and in particular, the fact is emphasized that the present monitoring system allows monitoring a blood component bag, thus enabling the certification of the collected data and ensuring a correct and safe association between the bag itself and a sensing device.

This is accomplished by the operation of the sensing device, in the operating position, in conjunction with the retaining means, in the closed configuration, wherein the bag cannot be removed without the sensing device keeping track of it.

Thus, the monitoring system also allows for the temporary removal of the sensing device from the bag.

Finally, the present monitoring system allows the detection of a plurality of different environmental conditions to which the bag is subjected, thanks to the presence of the humidity sensor and the brightness sensor integrated in the sensing device.

## Claims

1. Monitoring system (1) of blood component bags, comprising at least one sensing device (2) comprising at least one temperature sensor (3) configured to detect at least one value of temperature of at least one bag (S) containing blood components, said sensing device (2) being moveable with respect to said bag (S) between an operating position, wherein it is positioned at the point where said bag (S) is located and wherein said temperature sensor (3) detects said value of temperature, and a far-away position, wherein it is moved away from said bag (S), wherein the monitoring system comprises:
- at least one support (4) which is adapted to contain said bag (S) and comprising at least one housing (5) of said bag (S) and at least one seat (6) of said sensing device (2) in said operating position; and
- retaining means (7) of said bag (S) associated with said support (4) and positionable between an open configuration, wherein said bag (S) is movable with respect to said housing (5), and a closed configuration, wherein said bag (S) is retained inside said housing (5);
**characterized by** the fact that
said sensing device (2) comprises at least one contact sensor (11) configured to detect the positioning of said sensing device (2) in said operating position and by the fact that, in said operating position, said sensing device (2) is configured to lock said retaining means (7) in said closed configuration.

2. Monitoring system (1) according to claim 1, **characterized by** the fact that said retaining means (7) comprise a retaining element (12) movable with respect to said support (4) between said open configuration and said closed configuration.

3. Monitoring system (1) according to one or more of the preceding claims, **characterized by** the fact that said support (4) comprises a transit opening (8) adapted to allow the insertion/extraction of said bag (S) into/ from said support (4), in said closed configuration, said retaining element (12) being shaped to obstruct said transit opening (8).

4. Monitoring system (1) according to one or more of the preceding claims, **characterized by** the fact that said support (4) comprises an abutment portion (13) which is shaped to accommodate said retaining element (12) in said closed configuration.

5. Monitoring system (1) according to one or more of the preceding claims, **characterized by** the fact that said retaining element (12) is of substantially elongated conformation and comprises a first portion (14) associated with said support (4) and a second portion (15), opposite said first portion (14), shaped to engage with said abutment portion (13), in said closed configuration.

6. Monitoring system (1) according to one or more of the preceding claims, **characterized by** the fact that said retaining element (12) is movable in rotation around a relevant axis of rotation (R), at the point where said first portion (14) is located, between said open configuration and said closed configuration.

7. Monitoring system (1) according to one or more of the preceding claims, **characterized by** the fact that said seat (6) is defined at the point where said abutment portion (13) is located and by the fact that, in said operating position, said sensing device (2) is positioned at the point where said abutment portion (13) is located to prevent said retaining element (12) from rotating from said closed configuration to said open configuration.

8. Monitoring system (1) according to one or more of the preceding claims, **characterized by** the fact that said support (4) comprises anchoring means (18) of said sensing device (2) to said seat (6), of the interlocking type.

9. Monitoring system (1) according to one or more of the preceding claims, **characterized by** the fact that said sensing device (2) comprises an enclosure (21, 22) provided with a contact wall (21) facing a wall of said housing (5) at the point where said seat (6) is located, in said operating position, and an external wall (22) facing outwards, said temperature sensor (3) and said contact sensor (11) being arranged on said contact wall (21).

10. Monitoring system (1) according to one or more of the preceding claims, **characterized by** the fact that said sensing device (2) comprises at least one humidity sensor (23) configured to detect at least one value of humidity external to said bag (S) and at least one brightness sensor (24) configured to detect at least one value of brightness external to said bag (S).

11. Monitoring system (1) according to one or more of the preceding claims, **characterized by** the fact that said sensing device (2) comprises acquisition means (25) configured to acquire at least one identification control datum of a patient.

12. Monitoring system (1) according to one or more of the preceding claims, **characterized by** the fact that said sensing device (2) comprises at least one electronic unit (26) provided with a memory unit (27) configured to store at least one of either said value of temperature, said value of humidity, said value of brightness, at least one control datum or at least one preset datum.

13. Monitoring system (1) according to one or more of the preceding claims, **characterized by** the fact that said preset datum is selected out of: preset datum identifying said bag (S), preset datum identifying the contents of said bag (S), preset datum identifying a patient, preset datum identifying a destination of use of said bag (S).

14. Monitoring system (1) according to one or more of the preceding claims, **characterized by** the fact that said electronic unit (26) comprises a processing unit (28) configured to check the matching between said preset datum and a corresponding control datum, said sensing device (2) comprising indication means (29) configured to indicate a result of said check.

15. Monitoring system (1) according to one or more of the preceding claims, **characterized by** the fact that said sensing device (2) comprises transmission means (30) operationally connected to said memory unit (27) and configured to transmit at least one of either said values or at least one of said identification datum to at least one external unit (31).

## Patentansprüche

1. Überwachungssystem (1) von Blutkomponentenbeuteln, das mindestens eine Erfassungsvorrichtung (2) umfasst, die mindestens einen Temperatursensor (3) umfasst, der ausgebildet ist, mindestens einen Temperaturwert mindestens eines Beutels (S), der Blutkomponenten enthält, zu erfassen, wobei die Erfassungsvorrichtung (2) bezüglich des Beutels (S) zwischen einer Betriebsposition, in der sie an der Stelle positioniert ist, an der sich der Beutel (S) befindet wobei der Temperatursensor (3) den Temperaturwert erfasst, und einer entfernten Position beweglich ist, in der sie von dem Beutel (S) wegbewegt ist, wobei das Überwachungssystem umfasst:
- mindestens einen Träger (4), der ausgebildet ist, den Beutel (S) aufzunehmen, und der mindestens ein Gehäuse (5) für den Beutel (S) und mindestens einen Sitz (6) für die Erfassungsvorrichtung (2) in der Betriebsposition umfasst, und
- eine Halteeinrichtung (7) für den Beutel (S), die mit dem Träger (4) verbunden ist und zwischen einer offenen Konfiguration, in der der Beutel (S) bezüglich des Gehäuses (5) beweglich ist, und einer geschlossenen Konfiguration positionierbar ist, in der der Beutel (S) im Inneren des Gehäuses (5) gehalten ist,
**dadurch gekennzeichnet, dass**
die Erfassungsvorrichtung (2) mindestens einen Kontaktsensor (11) umfasst, der ausgebildet ist, die Positionierung der Erfassungsvorrichtung (2) in der Betriebsposition zu erfassen, und dadurch, dass die Erfassungsvorrichtung (2) in der Betriebsposition ausgebildet ist, die Halteeinrichtung (7) in der geschlossenen Konfiguration zu verriegeln.

2. Überwachungssystem (1) nach Anspruch 1, **dadurch gekennzeichnet, dass** die Halteeinrichtung (7) ein Halteelement (12) umfasst, das bezüglich des Trägers (4) zwischen der offenen Konfiguration und der geschlossenen Konfiguration beweglich ist.

3. Überwachungssystem (1) nach einem oder mehreren der vorhergehenden Ansprüche, **dadurch gekennzeichnet, dass** der Träger (4) eine Durchgangsöffnung (8) aufweist, die ausgebildet ist, das Einführen/ Entnehmen des Beutels (S) in den/ aus dem Träger (4) in der geschlossenen Konfiguration zu ermöglichen, wobei das Halteelement (12) geformt ist, die Durchgangsöffnung (8) zu versperren.

4. Überwachungssystem (1) nach einem oder mehreren der vorhergehenden Ansprüche, **dadurch gekennzeichnet, dass** der Träger (4) einen Anschlagteil (13) aufweist, der geformt ist, das Rückhalteelement (12) in der geschlossenen Konfiguration aufzunehmen.

5. Überwachungssystem (1) nach einem oder mehreren der vorhergehenden Ansprüche, **dadurch gekennzeichnet, dass** das Halteelement (12) eine im Wesentlichen längliche Form aufweist und einen ersten Abschnitt (14), der mit dem Träger (4) verbunden ist, und einen zweiten Abschnitt (15) umfasst, der dem ersten Abschnitt (14) gegenüberliegt und geformt ist, in der geschlossenen Konfiguration mit dem Anschlagabschnitt (13) in Eingriff zu treten.

6. Überwachungssystem (1) nach einem oder mehreren der vorhergehenden Ansprüche, **dadurch gekennzeichnet, dass** das Halteelement (12) um eine entsprechende Drehachse (R) an dem Punkt, an dem sich der erste Abschnitt (14) befindet, zwischen der offenen Konfiguration und der geschlossenen Konfiguration drehbar ist.

7. Überwachungssystem (1) nach einem oder mehreren der vorhergehenden Ansprüche, **dadurch gekennzeichnet, dass** der Sitz (6) an dem Punkt definiert ist, an dem sich der Anschlagteil (13) befindet, und dadurch, dass in der Betriebsposition die Erfassungsvorrichtung (2) an dem Punkt positioniert ist, an dem sich der Anschlagteil (13) befindet, um zu verhindern, dass sich das Halteelement (12) von der geschlossenen Konfiguration in die offene Konfiguration dreht.

8. Überwachungssystem (1) nach einem oder mehreren der vorhergehenden Ansprüche, **dadurch gekennzeichnet, dass** der Träger (4) Verankerungsmittel (18) der Erfassungsvorrichtung (2) an dem Sitz (6) vom Verriegelungstyp umfasst.

9. Überwachungssystem (1) nach einem oder mehreren der vorhergehenden Ansprüche, **dadurch gekennzeichnet, dass** die Erfassungsvorrichtung (2) ein Gehäuse (21, 22) umfasst, das mit einer Kontaktwand (21), die einer Wand des Gehäuses (5) an der Stelle zugewandt ist, an der sich der Sitz (6) in der Betriebsposition befindet, und einer Außenwand (22) versehen ist, die nach außen weist, wobei der Temperatursensor (3) und der Kontaktsensor (11) an der Kontaktwand (21) angeordnet sind.

10. Überwachungssystem (1) nach einem oder mehreren der vorhergehenden Ansprüche, **dadurch gekennzeichnet, dass** die Erfassungsvorrichtung (2) mindestens einen Feuchtigkeitssensor (23), der ausgebildet ist, mindestens einen Feuchtigkeitswert außerhalb des Beutels (S) zu erfassen, und mindestens einen Helligkeitssensor (24) umfasst, der ausgebildet ist, mindestens einen Helligkeitswert außerhalb des Beutels (S) zu erfassen.

11. Überwachungssystem (1) nach einem oder mehreren der vorhergehenden Ansprüche, **dadurch gekennzeichnet, dass** die Erfassungsvorrichtung (2) Erfassungsmittel (25) umfasst, die ausgebildet sind, mindestens einen Identifikationskontrolldatenwert eines Patienten zu erfassen.

12. Überwachungssystem (1) nach einem oder mehreren der vorhergehenden Ansprüche, **dadurch gekennzeichnet, dass** die Erfassungsvorrichtung (2) mindestens eine elektronische Einheit (26) umfasst, die mit einer Speichereinheit (27) versehen ist, die ausgebildet ist, mindestens einen der folgenden Werte zu speichern: entweder den Temperaturwert, den Feuchtigkeitswert, den Helligkeitswert, mindestens einen Steuerdatenwert oder mindestens einen voreingestellten Datenwert.

13. Überwachungssystem (1) nach einem oder mehreren der vorhergehenden Ansprüche, **dadurch gekennzeichnet, dass** der voreingestellte Datenwert ausgewählt ist aus: einem voreingestellten Datenwert, der den Beutel (S) identifiziert, einem voreingestellten Datenwert, der den Inhalt des Beutels (S) identifiziert, einem voreingestellten Datenwert, der einen Patienten identifiziert, einem voreingestellten Datenwert, der einen Verwendungsort des Beutels (S) identifiziert.

14. Überwachungssystem (1) nach einem oder mehreren der vorhergehenden Ansprüche, **dadurch gekennzeichnet, dass** die elektronische Einheit (26) eine Verarbeitungseinheit (28) umfasst, die ausgebildet ist, die Übereinstimmung zwischen dem voreingestellten Datenwert und einem entsprechenden Steuerdatenwert zu überprüfen, wobei die Erfassungsvorrichtung (2) Anzeigemittel (29) umfasst, die ausgebildet sind, ein Ergebnis der Überprüfung anzuzeigen.

15. Überwachungssystem (1) nach einem oder mehreren der vorhergehenden Ansprüche, **dadurch gekennzeichnet, dass** die Erfassungsvorrichtung (2) Übertragungsmittel (30) umfasst, die operativ mit der Speichereinheit (27) verbunden und ausgebildet sind, mindestens einen der Werte oder mindestens einen der Identifikationsdatenwerte an mindestens eine externe Einheit (31) zu übertragen.

## Revendications

1. - Système de surveillance (1) de poches de composants sanguins, comprenant au moins un dispositif de détection (2) comprenant au moins un capteur de température (3) configuré pour détecter au moins une valeur de température d'au moins une poche (S) contenant des composants sanguins, ledit dispositif de détection (2) étant déplaçable par rapport à ladite poche (S) entre une position de fonctionnement, dans laquelle il est positionné à l'endroit où ladite poche (S) est située et dans laquelle ledit capteur de température (3) détecte ladite valeur de température, et une position éloignée, dans laquelle il est éloigné de ladite poche (S), le système de surveillance comprenant :
- au moins un support (4) qui est adapté pour contenir ladite poche (S) et comprenant au moins un logement (5) de ladite poche (S) et au moins un siège (6) dudit dispositif de détection (2) dans ladite position de fonctionnement ; et
- des moyens de retenue (7) de ladite poche (S) associés audit support (4) et positionnables entre une configuration ouverte, dans laquelle ladite poche (S) est déplaçable par rapport audit logement (5), et une configuration fermée, dans laquelle ladite poche (S) est retenue à l'intérieur dudit logement (5) ;
**caractérisé par le fait que** ledit dispositif de détection (2) comprend au moins un capteur de contact (11) configuré pour détecter le positionnement dudit dispositif de détection (2) dans ladite position de fonctionnement et **par le fait que**, dans ladite position de fonctionnement, ledit dispositif de détection (2) est configuré pour verrouiller lesdits moyens de retenue (7) dans ladite configuration fermée.

2. - Système de surveillance (1) selon la revendication 1, **caractérisé par le fait que** lesdits moyens de retenue (7) comprennent un élément de retenue (12) déplaçable par rapport audit support (4) entre ladite configuration ouverte et ladite configuration fermée.

3. - Système de surveillance (1) selon une ou plusieurs des revendications précédentes, **caractérisé par le fait que** ledit support (4) comprend une ouverture de passage (8) adaptée pour permettre l'introduction/l'extraction de ladite poche (S) dans ledit support (4)/à partir dudit support (4), dans ladite configuration fermée, ledit élément de retenue (12) étant formé pour obstruer ladite ouverture de passage (8).

4. - Système de surveillance (1) selon une ou plusieurs des revendications précédentes, **caractérisé par le fait que** ledit support (4) comprend une partie de butée (13) qui est formée pour recevoir ledit élément de retenue (12) dans ladite configuration fermée.

5. - Système de surveillance (1) selon une ou plusieurs des revendications précédentes, **caractérisé par le fait que** ledit élément de retenue (12) est de conformation sensiblement allongée et comprend une première partie (14) associée audit support (4) et une seconde partie (15), à l'opposé de ladite première partie (14), formée pour s'engager avec ladite partie de butée (13), dans ladite configuration fermée.

6. - Système de surveillance (1) selon une ou plusieurs des revendications précédentes, **caractérisé par le fait que** ledit élément de retenue (12) est déplaçable en rotation autour d'un axe de rotation (R) approprié, à l'endroit où ladite première partie (14) est située, entre ladite configuration ouverte et ladite configuration fermée.

7. - Système de surveillance (1) selon une ou plusieurs des revendications précédentes, **caractérisé par le fait que** ledit siège (6) est défini à l'endroit où est située ladite partie de butée (13) et **par le fait que**, dans ladite position de fonctionnement, ledit dispositif de détection (2) est positionné à l'endroit où ladite partie de butée (13) est située pour empêcher ledit élément de retenue (12) de tourner de ladite configuration fermée à ladite configuration ouverte.

8. - Système de surveillance (1) selon une ou plusieurs des revendications précédentes, **caractérisé par le fait que** ledit support (4) comprend des moyens d'ancrage (18) dudit dispositif de détection (2) audit siège (6), du type à interverrouillage.

9. - Système de surveillance (1) selon une ou plusieurs des revendications précédentes, **caractérisé par le fait que** ledit dispositif de détection (2) comprend une enceinte (21, 22) comportant une paroi de contact (21) faisant face à une paroi dudit logement (5) à l'endroit où est situé ledit siège (6), dans ladite position de fonctionnement, et une paroi externe (22) tournée vers l'extérieur, ledit capteur de température (3) et ledit capteur de contact (11) étant disposés sur ladite paroi de contact (21).

10. - Système de surveillance (1) selon une ou plusieurs des revendications précédentes, **caractérisé par le fait que** ledit dispositif de détection (2) comprend au moins un capteur d'humidité (23) configuré pour détecter au moins une valeur d'humidité externe à ladite poche (S) et au moins un capteur de luminosité (24) configuré pour détecter au moins une valeur de luminosité externe à ladite poche (S).

11. - Système de surveillance (1) selon une ou plusieurs des revendications précédentes, **caractérisé par le fait que** ledit dispositif de détection (2) comprend des moyens d'acquisition (25) configurés pour acquérir au moins une donnée de contrôle d'identification d'un patient.

12. - Système de surveillance (1) selon une ou plusieurs des revendications précédentes, **caractérisé par le fait que** ledit dispositif de détection (2) comprend au moins une unité électronique (26) comportant une unité de mémoire (27) configurée pour stocker au moins l'une parmi ladite valeur de température, ladite valeur d'humidité, ladite valeur de luminosité, au moins une donnée de contrôle ou au moins une donnée prédéfinie.

13. - Système de surveillance (1) selon une ou plusieurs des revendications précédentes, **caractérisé par le fait que** ladite donnée prédéfinie est choisie parmi : une donnée prédéfinie identifiant ladite poche (S), une donnée prédéfinie identifiant le contenu de ladite poche (S), une donnée prédéfinie identifiant un patient, une donnée prédéfinie identifiant une destination d'utilisation de ladite poche (S).

14. - Système de surveillance (1) selon une ou plusieurs des revendications précédentes, **caractérisé par le fait que** ladite unité électronique (26) comprend une unité de traitement (28) configurée pour vérifier la correspondance entre ladite donnée prédéfinie et une donnée de contrôle correspondante, ledit dispositif de détection (2) comprenant des moyens d'indication (29) configurés pour indiquer un résultat de ladite vérification.

15. - Système de surveillance (1) selon une ou plusieurs des revendications précédentes, **caractérisé par le fait que** ledit dispositif de détection (2) comprend des moyens de transmission (30) connectés de manière opérationnelle à ladite unité de mémoire (27) et configurés pour transmettre au moins l'une parmi lesdites valeurs ou au moins une desdites données d'identification à au moins une unité externe (31).
